**Europäisches Patentamt**

**European Patent Office**

**Office européen des brevets**

(11) Publication number: **0 184 237**

**A1**

(12) # EUROPEAN PATENT APPLICATION

(21) Application number: **85201785.4**

(22) Date of filing: **01.11.85**

(51) Int. Cl.⁴: **A 61 F 11/00**

(30) Priority: **27.11.84 IT 2389684 U**

(43) Date of publication of application:
**11.06.86 Bulletin 86/24**

(84) Designated Contracting States:
**AT BE CH DE FR GB IT LI LU NL SE**

(71) Applicant: **S P M S.p.A**
**Via Provinciale, 22**
**I-21030 Roggiano di Brissago Valtr. (Varese)(IT)**

(72) Inventor: **Berutti, Giampiero**
**Via Privata Altipiano 25**
**I-21010 Porto Valtravaglia (Varese)(IT)**

(74) Representative: **Martegani, Franco et al,**
**Ingg. GUZZI e RAVIZZA S.r.l. Via Boccaccio, 24**
**I-20123 Milano(IT)**

(54) **Improved stick element for internal cleaning of the ears.**

(57) A stick element for internal cleaning of the ears comprises a substantially rigid handle (10) provided at one end with a tip (11) made of a soft material.

Said soft tip (11) can be a disposable piece inserted by slight forcing and in a freely removable manner into one end of said handle (10).

Fig. 1

EP 0 184 237 A1

"IMPROVED STICK ELEMENT FOR INTERNAL CLEANING OF THE EARS"

The present invention relates to a stick element designed so as to permit satisfactory cleaning of the ears without any risk of inavertently causing severe traumas in respect of the delicate inner parts of the ears.

As is known, the swab-sticks employed to date are entirely rigid and feature one or two extremities carrying a cotton swab or the like, said extremity or extremities being intended for introduction into the ear.

However, the cotton swab or the like fails to prevent traumas in respect if the very sensitive inner structures of the ear resulting from (and this is often the case) an unskilled use of the swab stick; nor does it provide a satisfactory cleaning action.

The object of the present invention is to obviate the problems mentioned above connected with the known swab sticks by embodying a stick element structured in such a way that even an unskilled use of it will not injure the subject whose ears are cleaned, especially neoneates and infants - who are particularly vulnerable in that their organs are at the development stage.

The stick element according to the invention comprises a substantially rigid handle with one of its ends provided with a tip made of a soft material.

The said soft tip can advantageously be a disposable piece and can be placed by slight forcing in a freely removable manner into one end of the rigid handle, which is therefore partially or wholly tubular.

The soft tip preferably has a round upper-portion and is separated by means of an intermediate baffle from a rear tang provided with annular ribbings, said tang being intended for insertion into one end of said handle, which is therefore tubular.

Another purpose served by the intermediate baffle is to prevent the tip from penetrating too deeply inside the ear.

The tip can be smooth or with perforations or can have a star-shaped cross-section (thus longitudinally ribbed), this last giving better cleaning efficiency.

The structural and functional characteristics of the invention and its advantages over the known art will become more apparent from an examination of the following description, referred to the appended drawings which show an example of a stick element for cleaning the ears embodied according to the innovative principles of the invention. In the enlarged drawings:

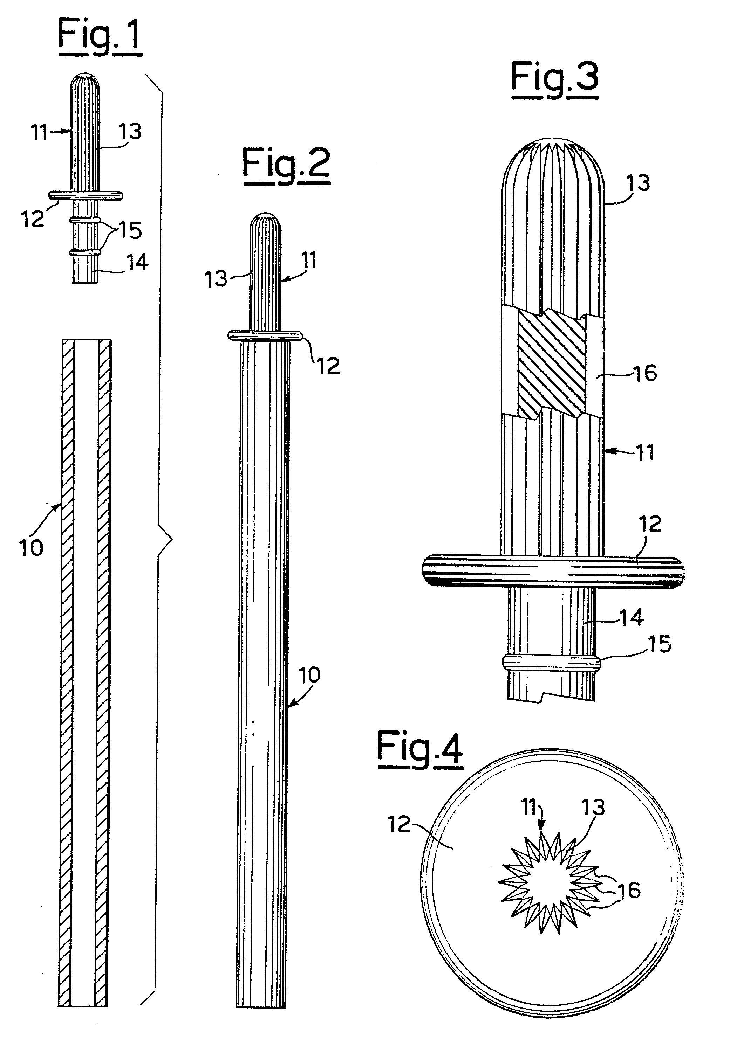

Figure 1 is an elarged view of a stick element according to the invention;
Figure 2 is a view of the same stick of Figure 1, but assembled;
Figure 3 is an elarged view of a particular of the tip; and
Figure 4 is a plan view of Figure 3.

With reference to the drawings, a stick element for cleaning the ears embodied according to the invention comprises a rigid tubular handle 10, for example made of plastic material, and a soft tip 11, for example made of rubber or expanded plastic material.

An intermediate baffle 12 separates the round upper portion 13 of said tip from a rear tang 14 featuring annular ribs 15, said tang being intended for insertion by slight forcing and in a freely removable manner into one end of the rigid tubular handle 10.

For preference, the upper-portion 13 has a star-shaped cross section, i.e. a series of longitudinal ribs 16 which facilitate the removal of dirt or cerumen from the external auditory meatus.

The upper-portion 13 can also carry a cotton swab covering or a perforated gauze which facilitates retention of cerumen or dirt and prevents it from accumulating at the end of the external meatus.

A stick element for cleaning the ears embodied according to the invention can be packaged in a pack (for example a blister pack) comprising a single recoverable handle 10 and a plurality of disposable tips 11. Each tip can advantageously be housed within its own seating from which it can be directly removed using the handle, thus hygienically. The pack will also comprise a seating for the handle, where it can be replaced after use.

0184237

CLAIMS

1) A stick element for internal cleaning of the ears comprising a substantially rigid handle provided at one end with a tip made of a soft material.

2) A stick element according to claim 1, in which said soft tip is a disposable piece inserted by slight forcing and in a freely removable manner into one end of said handle.

3) A stick element according to claim 2, in which said tip comprises a round upper-portion which is separated by an intermediate baffle from a rear tang provided with annular ribs and intended to be inserted into one end of said handle, which is therefore tubular.

4) A stick element according to claim 3, in which said round upper portion features a plurality of longitudinal ribs which give it a star-shaped cross-section.

Fig.1

Fig.2

Fig.3

Fig.4

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | US-A-1 980 826 (S. REISS)<br>* Figures 1,6 *<br>--- | 1-4 | A 61 F 11/00<br>A 61 F 13/20 |
| X | FR-A-2 249 640 (J. ROSSIGNOL)<br>* Whole document *<br>--- | 1-4 | |
| X | DE-A-2 648 725 (A. BOOG)<br>* Whole document *<br>--- | 1-3 | |
| X | FR-E- 58 319 (E. DESMAS)<br>* Figures 1,2 * | 1,2 | |
| A | <br>--- | 3 | |
| X | US-A-1 450 612 (F.A. SCHULZ)<br>* Whole document * | 1 | |
| | | | TECHNICAL FIELDS SEARCHED (Int Cl 4) |
| A | <br>--- | 2-4 | A 61 F |
| E | EP-A-0 158 543 (M. COLLIN)<br>* Abstract; figure 3A *<br><br>----- | 1 | |

The present search report has been drawn up for all claims

| Place of search | Date of completion of the search | Examiner |
|---|---|---|
| THE HAGUE | 04-03-1986 | WOLF C.H.S. |